# EUROPEAN PATENT SPECIFICATION

(11) **EP 0 308 065 B1**
(45) Date of publication and mention of the grant of the patent: **04.01.1995**
(21) Application number: 88307512.9
(22) Date of filing: 12.08.1988
(51) Int. Cl.: C07D 473/18, A61K 31/52

(54) **Therapeutic nucleosides**
Therapeutische Nucleoside
Nucléosides thérapeutiques

(30) Priority: 15.08.1987 GB 8719367
(43) Date of publication of application: 22.03.1989
(60) Divisional application: 93120604.9
(73) Proprietor: THE WELLCOME FOUNDATION LIMITED, London NW1 2BP (GB)
(72) Inventor: Beauchamp, Lilia Marie, Raleigh North Carolina 27607 (US)
(74) Representative: McMunn, Watson Palmer

(56) References cited:
- EP-A- 0 099 493
- JOURNAL OF MEDICINAL CHEMISTRY, vol. 26, no. 4, April 1983, pages 602-604, American Chemical Society, Columbus, Ohio, US; L. COLLA et al.: "Synthesis and antiviral activity of water-soluble esters of acyclovir [9-[(2-hydroxyethoxy)methyl]guanine]"
- JAMA, vol. 259, no. 3, January 15, 1988, pages 384-388

## Description

The invention relates to a new ester of 9-(2-hydroxyethoxymethyl)guanine having valuable antiviral properties.

9-(2-hydroxyethoxymethyl)guanine, otherwise known as acyclovir, possesses a potent antiviral activity, particularly against herpes viruses (H. J. Schaeffer et al, "Nature", 272 583-585 (1978), UK Patent Specification 1523865 and U.S. Patent Specification No. 4199574). Acyclovir is however only poorly soluble in water, thereby limiting the formulation of the drug in aqueous pharmaceutical preparations where solubility is required.

Also acyclovir is only poorly absorbed from the gastrointestinal tract after oral administration (15% recovery in the urine when tested in rats and 20% in humans). Such low bioavailability requires the administration of large doses of drug in order to achieve and maintain effective anti-viral levels in the plasma.

European Patent Specification 99493 and Journal of Medicinal Chemistry, vol. 26, no.4, 04/83, p.602-604 describe amino acid esters of acyclovir, specifically the glycine and alanine esters which show improved water-solubility compared with acyclovir.

We have now discovered that the L-valine ester of acyclovir, characterised by side-chain branching adjacent to the α-carbon atom, and which was not disclosed in European Patent Specification 99493 and the Journal of Medicinal Chemistry reference, surprisingly has improved bioavailability after oral administration compared with the alanine and glycine esters mentioned therein.

According to one feature of the present invention we provide the compound of formula (I) 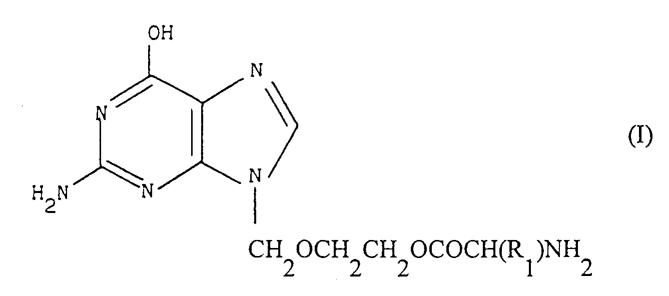
wherein R₁ represents a group of formula -CH[CH₃]₂ the ester group -OCOCH(R₁)NH₂ being in the L-configuration and pharmaceutically acceptable salts thereof. The compound of formula (I) can also be named as 2-((2-amino- 1,6-dihydro-6-oxo-9H-purin-9-yl)-methoxy)ethyl L-valinate.

In tests in rats, measuring the urinary recovery as acyclovir (% dose administered) after oral administration, the compounds of the invention show a large increase in absorption from the gut compared with the other esters and compared with acyclovir. This enables less drug to be administered while still providing equivalent drug levels in the plasma after oral absorption. The L-valinate compound is especially preferred by virtue of its particularly good absorption from the gut.

In addition to the relatively high bioavailability, the compound according to the invention possess substantially the same antiviral effect as acyclovir in vitro. The advantageous increase in bioavailability of the compound is thus not gained at the expense of antiviral potency. Indeed, it has been found that in certain clinical applications, e.g. the treatment of stromal keratitis, certain amino acid esters have been found to provide a superior therapeutic effect to acyclovir (EP 99493).

The pharmaceutically acceptable salts of the compound of formula (I) are preferably acid addition salts derived from an appropriate acid, e.g. hydrochloric, sulphuric, phosphoric, maleic, fumaric, citric, tartaric, lactic, acetic or p-toluenesulphonic acid. A particularly preferred salt is the hydrochloride salt of the compound of formula (I).

In experiments in animals, it was discovered that the oral administration of the compound of formula (I) above produced measurable levels of acyclovir in the plasma. Thus according to another aspect of the invention we provide a means of generating acyclovir in vivo by administration of the compound of formula (I) above or a pharmaceutically acceptable salt thereof to a mammal.

The compounds according to the invention may be prepared in conventional manner, e.g. by a process as described below.

Thus, according to a further feature of the present invention we provide a process for the preparation of the compound of formula (I) above and pharmaceutically acceptable salts thereof which comprises
a) reacting a compound of formula (II) 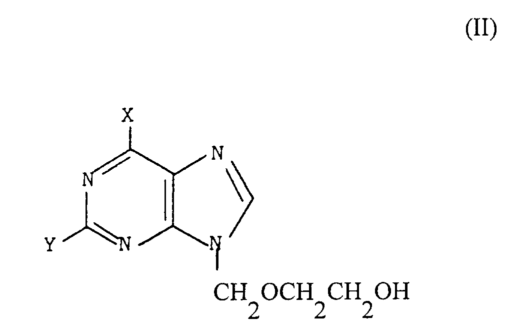 wherein X is an optionally protected hydroxy group, and Y is an optionally protected amino group with an optionally protected L-valine or a functional equivalent thereof;
b) converting a compound of formula (III) 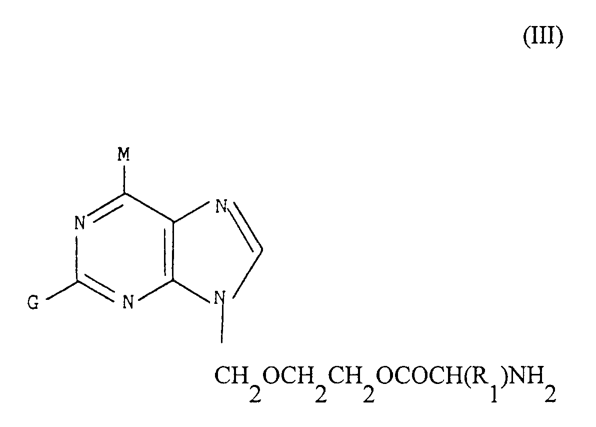 (wherein R₁ is as defined above; and M represents a hydroxy group and G represents an atom or group that can be replaced by or converted to an amino group; or G represents an amino group and M represents an atom or group that can be replaced by or converted to a hydroxy group) into the compound of formula (I) or a pharmaceutically acceptable salt thereof; or
c) reacting a compound of formula (IV) 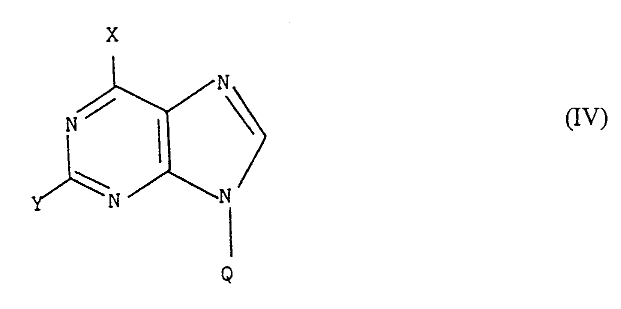 (wherein X and Y are as defined above and Q represents a leaving atom or group) with a compound of formula (V)

   ACH₂OCH₂CH₂OCOCH(R₁)R₂ (V)

   (wherein R₁ is as defined above, A represents a leaving group or atom and R² is an optionally protected amino group); and optionally effecting one or more of the following conversions, in any desired sequence:-
   i) removal of any protecting groups;
   ii) where the resulting product is a compound of formula (I), conversion of the said compound into a pharmaceutically acceptable salt thereof; and
   iii) where the resulting product is a pharmaceutically acceptable salt of a compound of formula (I), conversion of the said salt into the parent compound.

With regard to process a), the esterification reaction may be carried out in conventional manner, for example in a solvent such as pyridine or dimethylformamide in the presence of a coupling agent such as N,N'-dicyclohexylcarbodiimide, optionally in the presence of a catalytic base such as 4-dimethylaminopyridine. The water formed during the reaction may, if desired, be removed in conventional manner, for example by distillation or by the addition of a water-binding substance. Subsequently, the ester obtained as reaction product may be isolated in conventional manner.

As an alternative to the use of L-valine per se, a functional equivalent of the acid may be employed, e.g. an acid halide such as the acid chloride, or an acid anhydride. In such a case in order to avoid undesirable side-reactions, it is advantageous to use an amino-protected derivative. Examples of preferred amino-protecting groups including acyl, e.g. C₁₋₄alkanoyl such as acetyl and aryloxycarbonyl, e.g. benzyloxycarbonyl. A suitable amino-protected derivative, for example, is one wherein the amino group of the amino acid is replaced by an azido group.

Conversion of a compound of formula (III) into a compound of formula (I), by method b), can be achieved by various means. For example G may represent an azide group which can be reduced to an amino group by catalytic hydrogenation, using a suitable catalyst such as palladium on carbon. Alternatively, G may represent a halogen atom or an alkylthio or alkylsulphonyl group which can be converted to an azide group which in turn can be converted to an amino group by catalytic hydrogenation using, for example, hydrogen in the presence of palladium on carbon. For the preparation of the compound of formula (I), a compound of formula (III) wherein M is an amino group may be converted to a hydroxy group for example by treatment with a deaminating enzyme such as adenosine deaminase.

These processes together with other conventional processes are described in Fused Pyrimidines, Part II, Purines, Ed. by D.J. Brown (1971), Wiley-Interscience.

In process (c), the group Q in formula (IV) may, for example, represent a hydrogen atom; an acyl group, e.g. a C₁₋₄ alkanoyl group such as an acetyl group or an aroyl group such as a benzoyl group; or a tri-C₁₋₄alkylsilyl group such as a trimethylsilyl group. The group A in formula (V) may, for example, represent a halogen atom (e.g. chlorine) or an acyloxy group wherein the acyl moiety may be, for example, C₁₋₄alkanoyl group such as acetyl or an aroyl group such as benzoyl. The group R₂ may represent an amino-protecting group such as for example, C₁₋₄alkanoyl (e.g. acetyl) or aryloxycarbanoyl (e.g. benzyloxycarbonyl) and it may also represent an azido group. The reaction may be conveniently effected in a strong polar solvent such as dimethylformamide or hexamethylphosphoramide, advantageously in the presence of a base such as triethylamine or potassium carbonate. Alternatively, a thermal condensation may be effected by heating the compounds of formulae (IV) and (V) in the presence of a catalytic amount of a strong acid, e.g. sulphuric acid.

Compounds of formulae (II) to (V), employed as intermediates in the synthesis of the compound of formula (I), can be prepared in conventional manner, e.g. by procedures described in U.K. Patent Specification No. 1523865. These methods rely on intermediates prepared from simply substituted purines, which may be available commercially, or prepared according to techniques which are well known per se and which are disclosed in the literature such as the aforementioned text-book. Thus, for example, compounds of formula (III) may be generally prepared by using an analogous procedure to that of process (c), i.e. reacting an appropriate purine with a compound of formula (V).

The optional conversions i), ii) and iii) may be effected in conventional manner. Thus, for example, removal of protecting groups in conversion i) may be effected by hydrolysis, solvolysis or hydrogenolysis as appropriate. With regard to removal of protecting groups on the amino acid acyl radicals, hydrogenolysis, e.g. of aryloxycarbonyl protecting groups, and conversion of azido group, e.g. by catalytic hydrogenation, e.g. using a palladium catalyst, are preferred. With regard to protection of the groups in the 2- and/or 6-positions of the purine nucleus, these may be selected for example from arylmethyl groups e.g. benzyl; or tri-C₁₋₄alkylsilyl e.g. trimethylsilyl. Arylmethyl blocking groups may be removed for example by hydrogenolysis, e.g. by hydrogenation in the presence of Raney nickel or a palladium catalyst. Trialkylsilyl blocking groups may be removed for example by solvolysis e.g. by alcoholysis.

The conversion of a compound of formula (I) into a pharmaceutically acceptable salt may be effected in conventional manner, for example, by treatment of the compound with an appropriate acid to form an acid addition salt, for example, by lyophilsation of a methanolic solution of the parent ester with an acid solution.

Similarly, conversion of a salt into the parent compound of formula (I) may be effected in conventional manner.

The present invention also provides the compound of formula (I) and pharmaceutically acceptable salts thereof (hereinafter identified as "the active compounds") for use in medical therapy e.g. in the treatment or prophylaxis of a viral disease in an animal, e.g. a mammal such as man. The compounds are especially useful for the treatment or prophylaxis of diseases caused by various DNA viruses, such as herpes infections, for example herpes simplex, varicella zoster, cytomegalovirus as well as diseases caused by hepatitis B or Epstein-Barr viruses or human herpes virus -6 (HHV-6). The active compounds can also be used for the treatment or prophylaxis of retrovirus infections such as HIV infections and papilloma or wart virus infections. In addition to their use in human medical therapy, the compounds of formula (I) can be administered to other animals for treatment or prophylaxis of viral diseases, e.g. in other mammals. For example, the active compounds are especially useful for the treatment of equine rhinopneumonitis.

The present invention also provides a method for the treatment or prophylaxis of a viral disease in an animal, e.g. a mammal such as man, which comprises administering to the animal an effective antiviral amount of a compound of formula (I) or a pharmaceutically acceptable salt thereof.

The present invention also provides the use of a compound of formula (I) in the manufacture of a medicament for the treatment or prophylaxis of a viral infection.

The active compounds may be administered by any route appropriate to the condition to be treated, suitable routes including oral, rectal, nasal, topical (including buccal and sublingual) vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be as the discretion of the attendant physician or veterinarian. In general however, for each of these utilities and indications, a suitable effective dose will be in the range 0.1 to 250mg per kilogram bodyweight of recipient per day, preferably in the range 1 to 100mg per kilogram bodyweight per day and most preferably in the range 5 to 20mg per kilogram bodyweight per day; an optimum dose is about 10mg per kilogram bodyweight per day. (Unless otherwise indicated, all weights of active ingredient are calculated as the parent compound of formula (I): for salts thereof the figures would be increased proportionately). The desired dose is preferably presented as two, three, four or more sub-doses administered at appropriate intervals throughout the day. These sub-doses may be administered in unit dosage forms, for example, containing 10 to 1000mg, preferably 20 to 500mg and most preferably 100 to 400mg of active ingredient per unit dosage form.

The compounds of the present invention may be administered alone or in combination with other therapeutic agents, for example, with 9-(2-hydroxyethoxymethyl)guanine (acyclovir) used to treat herpes virus infections in particular HSV (I), and with zidovudine used to treat retroviral infections in particular HIV infections.

While it is possible for the active ingredients to be administered alone, it is preferably to present them as pharmaceutical formulations. The formulations, both for veterinary and for human use, of the present invention comprise at least one active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues e.g. mouth and skin, the formulations are preferably applied as a topical ointment or cream containing the active ingredient in an amount of for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base. In addition topical applications may be made transdermally by means of an iontophoretic device.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in a inert basis such as gelatin and glycerine, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described. Formulations for intramuscular administration are particularly preferred.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

For oral administration the compositions can be in the form of a tablet, granule, drench, paste, cachet, capsule or feed supplement. Granules may be made by the well known techniques of wet granulation, precompression or slugging. They can be administered to animals in an inert liquid vehicle so as to form a drench, or in a suspension with water or oil base. Preferably further accessory ingredients such as dispensing agent are included. These formulations preferably contain from 15 to 85% of the active ingredient.

The following Examples illustrate the present invention

### Example 1A

### 2-[(2-Amino-1,6-dihydro-6-oxo-9H-purin-9-yl)methoxy)ethyl L-valinate

### a) 2-[(2-Amino-1,6-dihydro-6-oxo-9H-purin-9-yl)methoxy]ethyl-N-[(benzyloxy)carbonyl] L-valinate

A suspension of acyclovir (2.000g; Burroughs Wellcome Co.) in dry dimethylformamide (DMF) (150ml) was warmed to 60°C to give a colorless solution. CBZ-L-valine (3.012g; Sigma Chemicals, St. Louis MO and J.Am.Chem.Soc, 79, 5697 (1957)), 4-dimethylaminopyridine (154mg; DMAP, Chem.Ber. 89 2921-33 [1956]) and dicyclohexylcarbodiimide (2.998g; DCC, US Patent 2656383) were added to the warm solution. The faint yellow solution was allowed to cool to room temperature and stirred overnight. A white precipitate was observed after 30 minutes. The reaction mixture was recharged with the above amounts of CBZ-L-valine, DMAP and DCC and the cloudy suspension stirred at room temperature for 2 days. The suspension was filtered to remove 1.418g, of a white solid. The colorless filtrate was concentrated to give a light yellow oil. The oil was purified by flash chromatography on silica gel, eluting with a methanol in dichloromethane gradient (0-15%) to yield the title compound as 3.751g (92.1%) of a white solid.

### b) 2-[(2-Amino-1,6-dihydro-6-oxo-9H-purin-9-yl)methoxy]ethyl L-valinate

A mixture of 2-[(2-amino-1,6-dihydro-6-oxo-9H-purin-9-yl)methoxy]ethyl N-[benzyloxy)carbonyl] L-valinate (5.0g), 5% palladium on carbon catalyst - 50% water (2g), and dimethylformamide (50ml), was shaken on a Parr apparatus under 40 psi H₂ for 3 hours. The reaction mixture was filtered through a pad of Celite and evaporated in vacuo to give an oil. A solid was crystallised from water/ethanol (1:3v/v) and recrystallised to yield 1.5g of title compound;

| | | | |
|---|---|---|---|
| Analysis Calcd. | C, 48.14; | H, 6.22; | N, 25.91 |
| Found | C, 47.84; | H, 6.26; | N, 25.75 |

### Example 1B

### 2-[2-Amino-1,6-dihydro-6-oxo-9H-purin-9-yl)methoxy]ethyl L-valinate hydrochloride monohydrate

### a) 2-[(2-Amino-1,6-dihydro-6-oxo-9H-purin-9-yl)methoxy]ethyl N-[(benzyloxy)carbonyl] L-valinate

A suspension of acyclovir (2.000g; Burroughs Wellcome Co.) in dry dimethylformamide (DMF) (150ml) was warmed to 60°C to give a colorless solution. CBZ-L-valine (3.012g; Sigma Chemicals, St. Louis MO and J.Am.Chem.Soc, 79, 5697 (1957)), 4-dimethylaminopyri- dine (154mg; DMAP, Chem.Ber. 89 2921-33 [1956]), and dicyclohexylcarbodiimide (2.998g; DCC, US 2656383) were added to the warm solution. The faint yellow solution was allowed to cool to room temperature and stirred overnight. A white precipitate was observed after 30 minutes. The reaction mixture was recharged with the above amounts of CBZ-L-valine, DMAP and DCC and the cloudy suspension stirred at room temperature for 2 days. The suspension was filtered to remove 1.418g, of a white solid. The colorless filtrate was concentrated to give a light yellow oil. The oil was purified by flash chromatography on silica gel, eluting with a methanol in dichloromethane gradient (0-15%) to yield the title compound as 3.751g (92.1%) of a white solid.

### b) 2-[(2-Amino-1,6-dihydro-6-oxo-9H-purin-9-yl)methoxy]ethyl L-valinate hydrochloride monohydrate

A mixture of 2-[(2-amino-1,6-dihydro-6-oxo-9H-purin-9-yl)methoxy]ethyl N-[benzyloxy)carbonyl] L-valinate, (3.730g), 5% palladium on carbon catalyst (377mg), methanol (100ml), tetrahydrofuran (THF) (100ml) and a 0.5 M aqueous HCl solution (18ml) was shaken on a Parr apparatus under 60 psi H₂ for one day. The reaction mixuture was filtered through a pad of Celite then concentrated to give a white solid. This solid was recrystallized from water/ethanol to yield the title compound as 1.762g (60.0%) of a white powder; mp 150°C (solid shrinks, gradually changes to oil and decomposes with foaming at 195°C).

| | | | | |
|---|---|---|---|---|
| Analysis Calcd. | C, 41.22; | H, 6.12; | N, 22.19; | Cl, 9.36 |
| Found | C, 41.09; | H, 6.10; | N, 22.12; | Cl, 9.28 |

### Example 2: Tablet Formulations

The following formulations A, B and C are prepared by wet granulation of the ingredients with a solution of providone, followed by addition of magnesium stearate and compression.

### Formulation A

| | mg/tablet | mg/tablet |
|---|---|---|
| Active Ingredient | 250 | 250 |
| Lactose B.P. | 210 | 26 |
| Povidone B.P. | 15 | 9 |
| Sodium Starch Glycollate | 20 | 12 |
| Magnesium Stearate | 5 | 3 |
| | 5̅0̅0̅ | 3̅0̅0̅ |

### Formulation B

| | mg/tablet | mg/tablet |
|---|---|---|
| Active Ingredient | 250 | 250 |
| Lactose | 150 | - |
| Avicel PH 101 | 60 | 26 |
| Povidone B.P. | 15 | 9 |
| Sodium Starch Glycollate | 20 | 12 |
| Magnesium Stearate | 5 | 3 |
| | 5̅0̅0̅ | 3̅0̅0̅ |

### Formulation C

| | mg/tablet |
|---|---|
| Active Ingredient | 100 |
| Lactose | 200 |
| Starch | 50 |
| Povidone B.P. | 5 |
| Magnesium Stearate | 4 |
| | 3̅5̅9̅ |

The following formulations D and E, are prepared by direct compression of the admixed ingredients. The lactose in formulation E is of the compression type.

### Formulation D

| | mg/tablet |
|---|---|
| Active Ingredient | 250 |
| Avicel | 150 |
| Magnesium Stearate | 4 |
| | 4̅0̅4̅ |

### Formulation E

| | mg/tablet |
|---|---|
| Active Ingredient | 250 |
| Lactose | 150 |
| Avicel | 100 |
| Magnesium Stearate | 5 |
| | 5̅0̅5̅ |

### Formulation F (Controlled Release Formulation)

The formulation is prepared by wet granulation of the ingredients (below) with a solution of povidone followed by the addition of magnesium stearate and compression.

| | mg/tablet |
|---|---|
| Active Ingredient | 500 |
| Hydroxypropylmethylcellulose (Methocel K4M Premium) | 112 |
| Lactose B.P. | 53 |
| Povidone B.P. | 28 |
| Magnesium Stearate | 7 |
| | 7̅0̅0̅ |

### Example 3: Capsule Formulations

### Formulation A

A capsule formulation is prepared admixing the ingredients of Formulation D in Example 2 above and filling into a two-part hard gelatin capsule. Formulation B (infra) is prepared in a similar manner.

### Formulation B

| | mg/capsule |
|---|---|
| Active Ingredient | 250 |
| Lactose B.P. | 143 |
| Sodium Starch Glycollate | 25 |
| Magnesium Stearate | 2 |
| | 4̅2̅0̅ |

### Formulation C

| | mg/capsule |
|---|---|
| Active Ingredient | 250 |
| Macrogol 4000 B.P. | 350 |
| | 6̅0̅0̅ |

Capsules are prepared by melting the Macrogol 4000 BP, dispersing the active ingredient in the melt and filling the melt into a two-part hard gelatin capsule.

### Formulation D

| | |
|---|---|
| Active Ingredient | 250 |
| Lecithin | 100 |
| Arachis Oil | 100 |
| | 4̅5̅0̅ |

Capsules are prepared by dispersing the active ingredient in the lecithin and arachis oil and filling the dispersion into soft, elastic gelatin capsules.

### Formulation E (Controlled Release Capsule

The following controlled release capsule formulation is prepared by extruding ingredients a, b and c using an extruder, followed by spheronisation of the extrudate and drying. The dried pellets are then coated with release-controlling membrane (d) and filled into a two-piece, hard gelatin capsule.

| | mg/capsule |
|---|---|
| Active Ingredient | 250 |
| Microcrystalline Cellulose | 125 |
| Lactose B.P. | 125 |
| Ethyl Cellulose | 13 |
| | 5̅1̅3̅ |

### Example 4: Ophthalmic Solution

| | |
|---|---|
| Active Ingredient | 0.5 |
| Propylene Glycol | 0.2 g |
| Thiomersal | 0.001g |
| Purified water to pH adjusted to 7.5 | 100 ml |

### Example 5: Injectable Formulation

| | |
|---|---|
| Active Ingredient | 0.200 g |
| Sterile, pyrogen free citrate buffer (pH 7.0) to | 10 ml |

The active ingredient is dissolved in most of the citrate buffer (35°-40°C), then made up to volume and filtered through a sterile micropore filter into a sterile 10ml amber glass vial (type 1) and sealed with sterile closures and overseals.

### Example 6: Intramuscular Injection

| | |
|---|---|
| Active Ingredient | 0.20g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3ml. The mixture is then filtered through a sterile micropore filter and sealed in sterile 3ml amber glass vials (type 1).

### Example 7: Syrup Suspension

| | |
|---|---|
| Active Ingredient | 0.25 g |
| Sorbitol Solution | 1.50 g |
| Glycerol | 2.00 g |
| Sodium Benzoate | 0.005 g |
| Flavour, | 0.0125 ml |
| Purified Water q.s. to | 5.00 ml |

The sodium benzoate is dissolved in a portion of the purified water and the sorbitol solution added. The active ingredient is added and dissolved. The glycerol and flavours are added and mixed in. Water is added to a final volume of 5ml.

### Example 8: Suppository

| | |
|---|---|
| Active Ingredient (63µm)* | 250 |
| Hard Fat, BP (Witepsol H25 - Dynamit NoBel) | 1700 |
| | 1̅9̅5̅0̅ |

| | |
|---|---|
| * The active ingredient is used as a powder wherein at least 90% of the particles are of 63µm diameter or less. | |

One-fifth of the Witepsol H15 is melted in a steam-jacketed pan at 45°C maximum. The active ingredient is sifted through a 200µm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250µm stainless steel screen and, with continuous stirring, is allowed to cool to 40°C. At a temperature of 38°C to 40°C, 2.02g of the mixture is filled into suitable plastic moulds. The suppositories are allowed to cool to room temperature.

### Example 9: Pessaries

| | |
|---|---|
| Active Ingredient (63 µm) | 250 |
| Anhydrous Dextrose | 543 |
| Starch | 200 |
| Magnesium Stearate | 7 |
| | 1̅0̅0̅0̅ |

The above ingredients are mixed directly and pessaries prepared by direct compression of the resulting mixture.

### Example 10

### a) Antiviral Activity

Herpes Simplex Virus (HSV 1) was assayed in monolayers of Vero cells in multiwell trays. Activity of compounds was determined in the plaque reduction assay, in which a cell monolayer was infected with a suspension of HSV 1, and then overlaid with nutrient agarose in the form of a gel to ensure that there was no spread of virus throughout the culture. A range of concentrations of compound of known molarity was incorporated in the nutrient agarose overlay. Plaque numbers at each concentration were expressed as percentages of the control and a dose-response curve was drawn. From this curve the 50% inhibitory concentration (IC₅₀) was estimated.

| Compound | IC₅₀µM |
|---|---|
| Example 1B | 0.84 |
| Acyclovir | 0.08 - 0.1 |

### b) Determination of Oral Bioavailability

Long Evans Rats were administered the compound to be tested by gavage at a dose equivalent to 25mg/kg acyclovir. The urine was collected for 24 and 48 hours post-dose, ultrafiltered, and analysed by reverse-phase high-pressure liquid chromatography. The oral bioavailability of the compound was expressed as the percent of the dose excreted in the urine as acyclovir.

| Compound | Urinary Recovery (% of dose) as acyclovir |
|---|---|
| Example 1B | 63 |
| Acyclovir (ACV) | 15 |
| Glycyl ester of ACV | 30 |
| L-alanyl ester of ACV | 34 |

### d) Toxicity Data

### Determination of Growth Inhibition of Uninfected Mammalian Cells

The capability of candidate compounds to inhibit the growth of D98 cells (human) and L cells (murine) was measured by determination of cell number following three days exposure of a standard number of cells to various dilutions of compound (Rideout, J.L., Krenitsky, T.A. Koszalka, G.W., Cohn, N.K., Chao, E.Y., Elion, G.B., Latter, V.S., and Williams, R.B. (1982) J.Med.Chem. 25: 1040-1044). The cell number was then compared to the number obtained in the absence of compound. Cell enumeration was performed by either direct particle counts following trypsinization of the monolayer, or by spectrophotometric determination of the amount of vital stain taken up by the cells. Comparable results were obtained with both methods.

### Data Analysis

The concentration of compound resulting in 50% of control values (IC50) was calculated either by direct interpolation from graphs of the log of the compound concentration versus the percent of control value, or from a computer program which analyses the data according to the same algorithm. Data in the range of 20% to 80% of control were used in these calculations.

| Example | Cell Toxicity (% of control at 100µm) | |
|---|---|---|
| | D-98 Cells | L-Cells |
| ACV (acyclovir) | 99 | 72 |
| 1B | 91 | 85 |

## Claims (Claims for the following Contracting State(s): AT, BE, CH, DE, FR, GB, IT, LI, LU, NL, SE)

1. 2-((2-Amino-1,6-dihydro-6-oxo-9H-purin-9-yl)methoxy)ethyl L-valinate or a pharmaceutically acceptable salt thereof.

2. A salt of a compound according to claim 1 wherein the salt is an acid addition salt.

3. A salt of a compound according to claim 2 which is derived from hydrochloric, sulphuric, phosphoric, maleic, fumaric, citric, tartaric, lactic, acetic or p-toluenesulphonic acid.

4. 2-((2-Amino-1,6-dihydro-6-oxo-9H-purin-9-yl)methoxy)ethyl L-valinate hydrochloride.

5. 2-((2-Amino-1,6-dihydro-6-oxo-9H-purin-9-yl)methoxy)ethyl L-valinate.

6. A compound according to any of the preceding claims for use in medical therapy.

7. A compound according to claim 6 for use in the treatment or prophylaxis of a viral infection.

8. A compound according to claim 7 for use in the treatment or prophylaxis of a herpes simplex virus infection.

9. A compound according to claim 8 for use in the treatment or prophylaxis of a herpes simplex virus type 1 infection.

10. A compound according to claim 7 for use in the treatment or prophylaxis of a varicella zoster virus infection.

11. A compound according to claim 10 for use in the treatment or prophylaxis of varicella.

12. A compound according to claim 10 for use in the treatment or prophylaxis of zoster.

13. A compound according to claim 9 for use in the treatment or prophylaxis of a cytomegalovirus infection.

14. A compound according to claim 7 for use in the treatment or prophylaxis of an Epstein-Barr virus infection.

15. A compound according to claim 7 for use in the treatment or prophylaxis of a hepatitis B virus infection.

16. Use of a compound according to any claims 1 to 5 in the manufacture of a medicament for the treatment or prophylaxis of a viral infection.

17. Use of a compound according to any of claims 1 to 5 in the manufacture of a medicament for the treatment or prophylaxis of a herpes viral infection.

18. Use of a compound according to any of claims 1 to 5 in the manufacture of a medicament for the treatment or prophylaxis of a herpes simplex virus infection.

19. Use of a compound according to claim 18 in the manufacture of a medicament for the treatment or prophylaxis of a herpes simplex type 1 infection.

20. Use of a compound according to any of claims 1 to 5 in the manufacture of a medicament for the treatment or prophylaxis of a varicella zoster virus infection.

21. Use of a compound according to claim 20 in the manufacture of a medicament for the treatment or prophylaxis of varicella.

22. Use of a compound according to claim 20 in the manufacture of a medicament for the treatment or prophylaxis of zoster.

23. Use of a compound according to any of claims 1 to 5 in the manufacture of a medicament for the treatment or prophylaxis of a cytomegalovirus infection.

24. Use of a compound according to any of claims 1 to 5 in the manufacture of a medicament for the treatment or prophylaxis of an Epstein-Barr virus infection.

25. Use of a compound according to any one of claims 1 to 5 in the manufacture of a medicament for the treatment or prophylaxis of a hepatitis B virus infection.

26. A pharmaceutical composition comprising as active ingredient a compound according to any of claims 1 to 5 together with a pharmaceutically acceptable carrier.

27. A pharmaceutical composition according to claim 26 which is adapted for use in the treatment or prophylaxis of a herpes simplex virus infection.

28. A pharmaceutical composition according to claim 26 which is adapted for use in the treatment or prophylaxis of a varicella zoster virus infection.

29. A pharmaceutical composition according to claim 26 which is adapted for use in the treatment or prophylaxis of a cytomegalovirus infection.

30. A pharmaceutical composition according to any of claims 26 to 29 adapted for oral administration.

31. A pharmaceutical composition according to claim 30 in the form of a tablet or capsule.

32. A pharmaceutical composition according to claim 31 wherein the tablet or capsule contains from 10 to 1000mg of a compound according to any of claims 1 to 5.

33. A pharmaceutical composition according to claim 31 wherein the tablet or capsule contain from 20 to 500 mg of a compound according to any of claims 1 to 5.

34. Use of the compounds according to claims 1 to 5 in combination with zidovudine in the manufacture of a medicament for use in the treatment or prophylaxis of HIV infection.

35. A process for the preparation of a compound of formula (I) 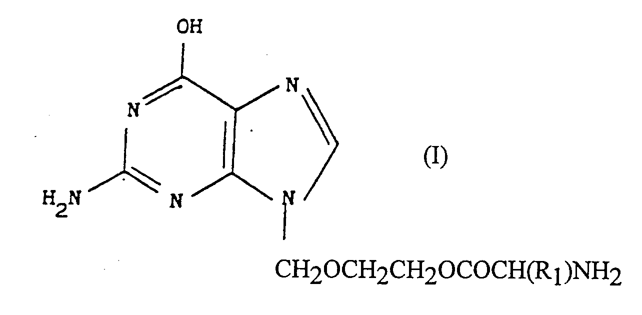 wherein R₁ represents a group of formula -CH[CH₃]₂ the ester group (OCOCH(R₁)NH₂) being in the L-configuration, or a pharmaceutically acceptable salt thereof which comprises:
a) reacting a compound of formula (II) 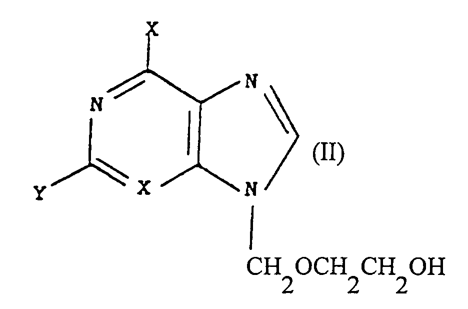 wherein X is an optionally protected hydroxy group, and Y is an optionally protected amino group with an optionally protected L-valine or a functional equivalent thereof;
b) converting a compound of formula (III) 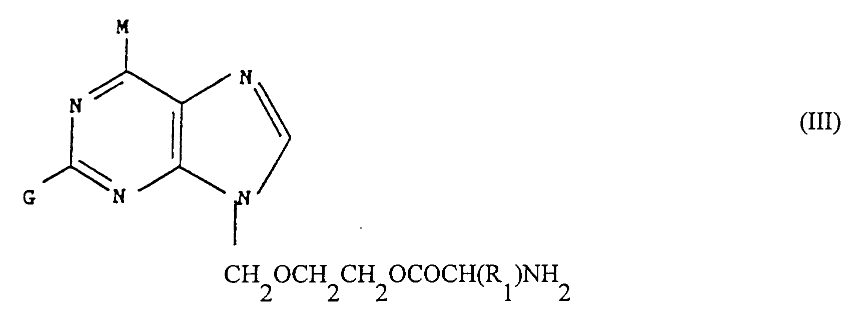 wherein R₁ is as defined above; and M represents a hydroxy group and G represents an atom or group that can be replaced by or converted to an amino group; or G represents an amino group and M represents an atom or group that can be replaced by or converted to a hydroxy group, into a compound of formula (I) or a pharmaceutically acceptable salt thereof; or
c) reacting a compound of formula (IV) 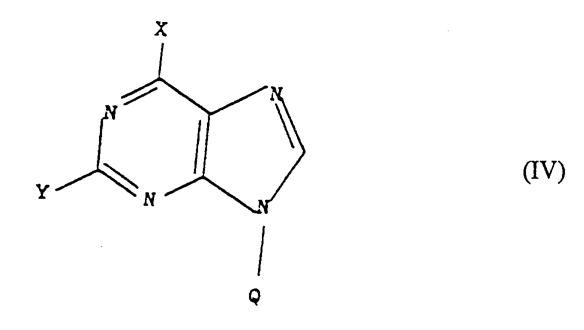 wherein X and Y are as defined above and Q represents a leaving atom or group with a compound of formula (V)
ACH₂OCH₂CH₂OCOCH(R₁)R₂ (V)
wherein R₁ is as defined above, A represents a leaving group or atom and R₂ is an optionally protected amino group; and optionally effecting one or more of the following conversions, in any desired sequence:-
i) removal of any protecting groups;
ii) where the resulting product is a compound of formula (I), conversion of the said compound into a pharmaceutically acceptable salt thereof; and
iii) where the resulting product is a pharmaceutically acceptable salt of a compound of formula (I), conversion of the said salt into the parent compound.

## Claims (Claims for the following Contracting State(s): ES, GR)

1. A process for the preparation of a compound of formula (I) 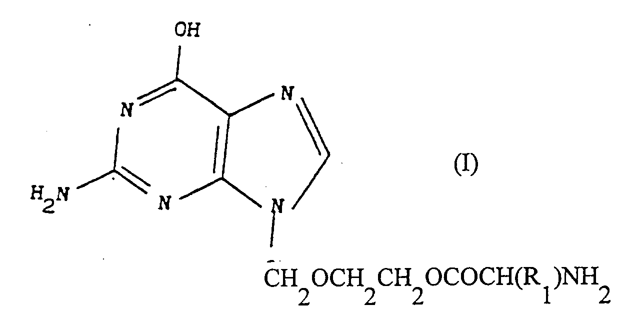 wherein R₁ represents a group of formula -CH[CH₃]₂, the ester group (OCOCH(R₁)NH₂) being in the L-configuration or a pharmaceutically acceptable salt thereof which comprises:
a) reacting a compound of formula (II) 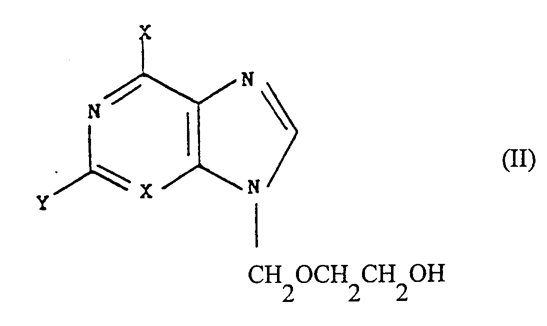 wherein X is an optionally protected hydroxy group, and Y is an optionally protected amino group with an optionally protected L-valine or a functional equivalent thereof;
b) converting a compound of formula (III) 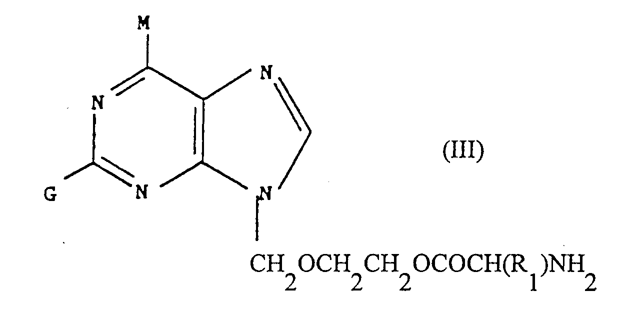 wherein R₁ is as defined in claim 1; and M represents a hydroxy group and G represents an atom or group that can be replaced by or converted to an amino group; or G represents an amino group and M represents an atom or group that can be replaced by or converted to a hydroxy group, into a compound of formula (I) or a pharmaceutically acceptable salt thereof; or
c) reacting a compound of formula (IV) 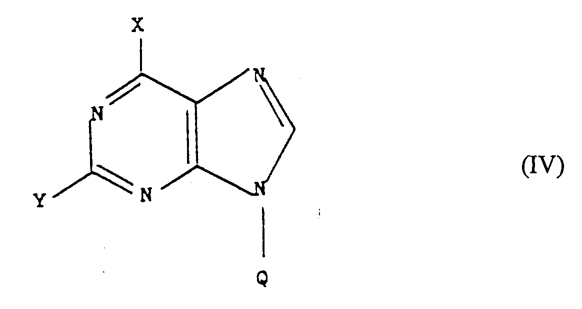 wherein X and Y are as defined above and Q represents a leaving atom or group with a compound of formula (V)
ACH₂OCH₂CH₂OCOCH(R₁)R₂ (V)
wherein R₁ is as defined in claim 1, A represents a leaving group or atom and R₂ is an optionally protected amino group; and optionally effecting one or more of the following conversions, in any desired sequence:-
i) removal of any protecting groups;
ii) where the resulting product is a compound of formula (I), conversion of the said compound into a pharmaceutically acceptable salt thereof; and
iii) where the resulting product is a pharmaceutically acceptable salt of a compound of formula (I), conversion of the said salt into the parent compound.

2. A process according to claim 1 wherein the compound of formula (I) is 2-((2-Amino-1,6-dihydro-6-oxo-9H-purin-9-yl)methoxy) ethyl L-valinate.

3. A process for the preparation of a salt of a compound according to claims 1 or 2 which comprises mixing a compound of formula (I) with an acid to form a salt.

4. A process according to claim 3 wherein said acid is selected from the group consisting of hydrochloric acid, sulphuric acid, phosphoric acid, maleic acid, fumaric acid, citric acid, tartaric acid, lactic acid, acetic acid and p-toluenesulphonic acid.

5. A process of claim 4 wherein said acid is hydrochloric acid.

6. A process according to any of the preceding claims wherein the compound of formula (I) is 2-((2-Amino-1,6-dihydro-6-oxo-9H-purin-9-yl)- methoxy) ethyl L-valinate hydrochloride monohydrate.

7. A process according to any of the preceding claims wherein the protecting group in process A of claim 1 is N,N'-dicyclohexylcarbodiimide.

8. A process according to claim 7 which is carried out in pyridine or dimethylformamide.

9. A process according to claim 7 or 8 which is carried out in the presence of a catalytic base.

10. A process according to claim 9 wherein the catalytic base is 4-dimethylaminopyridine.

11. A process according to any of the preceding claim wherein the functional derivative of process A is an acid halide or acid anhydride wherein the amino group of the L-valine is protected.

12. A process according to claim 11 wherein the amino protecting group is an acetyl or an aryloxycarbonyl.

13. A process according to process B of any of the preceding claims 1 to 6 wherein G represents an azide group which is reduced to an amino group by catalytic hydrogenation.

14. A process according to process B of any of claims 1 to 6 wherein G represents a halogen atom or an alkylthio or alkylsulphonyl group which is converted to an azide group which is in turn converted to an amino group by catalytic hydrogenation.

15. A process according to claims 13 or 14 wherein M is an amino group and is converted to a hydroxy group by treatment with a deaminating enzyme.

16. A process according to process C of any of claims 1 to 6 wherein Q represents a hydrogen atom, an acyl group or a tri-C₁₋₄ alkylsilyl group.

17. A process according to claim 16 wherein Q represents an acetyl or benzoyl group.

18. A process according to claims 15 or 16 wherein A represents a halogen or acyloxy group wherein the acyl moiety is a C₁₋₄ alkanoyl or an aroyl group

19. A process according to any of claims 16 to 18 wherein R² represents C₁₋₄ alkanoyl, aryloxycarbonyl or an azido group.

20. A process according to claims 16 to 19 which is effected in dimethylformamide or hexamethylphosphoramide in the presence of a base.

21. A process according to claims 16 to 18 wherein a thermal condensation is effected by heating the compounds of formula (IV) and (V) in the presence of a catalytic amount of strong acid.

22. A process according to any one of the preceding claim wherein the protecting groups are removed by hydrolysis, solvolysis or hydrogenolysis.

23. A process according to claim 22 wherein when the protecting group is an aryloxycarbonyl, hydrogenolysis is used to remove it.

24. A process for the preparation of a pharmaceutical formulation containing a compound of formula (I) as defined in claim 1 or a pharmaceutically acceptable salt thereof which comprises preparing a compound of formula (I) or a pharmaceutically acceptable salt thereof by:
a) reacting a compound of formula (II) 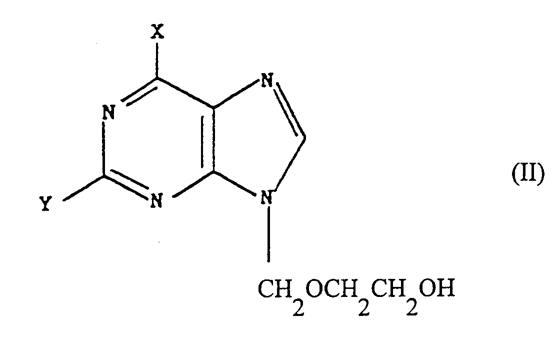 wherein X is an optionally protected hydroxy group, and Y is an optionally protected amino group with an optionally protected L-valine or a functional equivalent thereof;
b) converting a compound of formula (III) 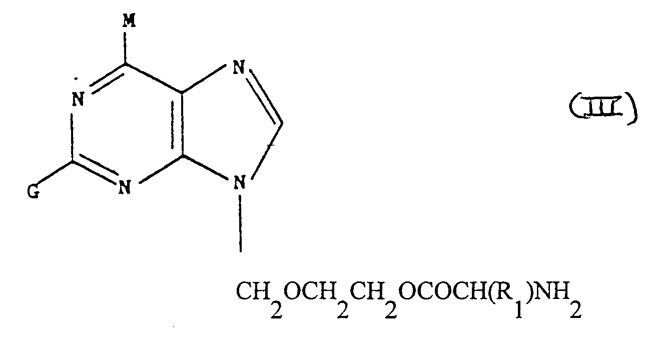 wherein R₁ is as defined in claim 1; and M represents a hydroxy group and G represents an atom or group that can be replaced by or converted to an amino group; or G represents an amino group and M represents an atom or group that can be replaced by or converted to a hydroxy group, into a compound of formula (I) or a pharmaceutically acceptable salt thereof; or
c) reacting a compound of formula (IV) 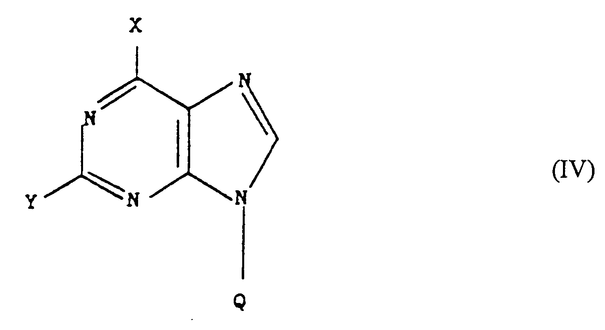 wherein X and Y are as defined above and Q represents a leaving atom or group with a compound of formula (V)
ACH₂OCH₂CH₂OCOCH(R₁)R₂ (V)
wherein R₁ is as defined in claim 1, A represents a leaving group or atom and R₂ is an optionally protected amino group; and optionally effecting one or more of the following conversions, in any desired sequence:-
i) removal of any protecting groups;
ii) where the resulting product is a compound of formula (I), conversion of the said compound into a pharmaceutically acceptable salt thereof; and
iii) where the resulting product is a pharmaceutically acceptable salt of a compound of formula (I), conversion of the said salt into the parent compound;
and bring the resulting compound into association with a pharmaceutically acceptable carrier.

## Patentansprüche (Patentansprüche für folgende(n) Vertragsstaat(en): AT, BE, CH, DE, FR, GB, IT, LI, LU, NL, SE)

1. 2-((2-Amino-1,6-dihydro-6-oxo-9H-purin-9-yl]methoxy]ethyl-L-valinat oder ein pharmazeutisch annehmbares Salz davon.

2. Salz einer Verbindung nach Anspruch 1, wobei das Salz ein Säureadditionssalz ist.

3. Salz einer Verbindung nach Anspruch 2, welches aus Chlorwasserstoff-, Schwefel-, Phosphor-, Malein-, Fumar-, Zitronen-, Wein-, Milch-, Essig- oder p-Toluolsulfonsäure abgeleitet ist.

4. 2-((2-Amino-1,6-dihydro-6-oxo-9H-purin-9-yl)methoxy)ethyl-L-valinathydrochlorid.

5. 2-((2-Amino-1,6-dihydro-6-oxo-9H-purin-9-yl)methoxy)ethyl-L-valinat.

6. Verbindung nach mindestens einem der vorangehenden Ansprüche zur Verwendung bei der medizinischen Therapie.

7. Verbindung nach Anspruch 6 zur Verwendung bei der Behandlung oder Prophylaxe einer Virusinfektion.

8. Verbindung nach Anspruch 7 zur Verwendung bei der Behandlung oder Prophylaxe einer Herpes simplex-Virusinfektion.

9. Verbindung nach Anspruch 8 zur Verwendung bei der Behandlung oder Prophylaxe einer Herpes simplex-Virustyp 1-Infektion.

10. Verbindung nach Anspruch 7 zur Verwendung bei der Behandlung oder Prophylaxe einer Varicella zoster-Virusinfektion.

11. Verbindung nach Anspruch 10 zur Verwendung bei der Behandlung oder Prophylaxe von Varicella.

12. Verbindung nach Anspruch 10 zur Verwendung bei der Behandlung oder Prophylaxe von Gürtelrose.

13. Verbindung nach Anspruch 7 zur Verwendung bei der Behandlung oder Prophylaxe einer Zytomegalovirusinfektion.

14. Verbindung nach Anspruch 7 zur Verwendung bei der Behandlung oder Prophylaxe einer Epstein-Barr-Virusinfektion.

15. Verbindung nach Anspruch 7 zur Verwendung bei der Behandlung oder Prophylaxe einer Hepatitis B-Virusinfektion.

16. Verwendung einer Verbindung nach mindestens einem der Ansprüche 1 bis 5 bei der Herstellung eines Arzneimittels zur Behandlung oder Prophylaxe einer Virusinfektion.

17. Verwendung einer Verbindung nach mindestens einem der Ansprüche 1 bis 5 bei der Herstellung eines Arzneimittels zur Behandlung oder Prophylaxe einer Herpesvirusinfektion.

18. Verwendung einer Verbindung nach mindestens einem der Ansprüche 1 bis 5 bei der Herstellung eines Arzneimittels zur Behandlung oder Prophylaxe einer Herpes simplex-Virusinfektion.

19. Verwendung einer Verbindung nach Anspruch 18 bei der Herstellung eines Arzneimittels zur Behandlung oder Prophylaxe einer Herpes simplex-Typ 1-Infektion.

20. Verwendung einer Verbindung nach mindestens einem der Ansprüche 1 bis 5 bei der Herstellung eines Arzneimittels zur Behandlung oder Prophylaxe einer Varicella zoster-Virusinfektion.

21. Verwendung einer Verbindung nach Anspruch 20 bei der Herstellung eines Arzneimittels zur Behandlung oder Prophylaxe von Varicella.

22. Verwendung einer Verbindung nach Anspruch 20 bei der Herstellung eines Arzneimittels zur Behandlung oder Prophylaxe von Gürtelrose.

23. Verwendung einer Verbindung nach mindestens einem der Ansprüche 1 bis 5 bei der Herstellung eines Arzneimittels zur Behandlung oder Prophylaxe einer Zytomegalovirusinfektion.

24. Verwendung einer Verbindung nach mindestens einem der Ansprüche 1 bis 5 bei der Herstellung eines Arzneimittels zur Behandlung oder Prophylaxe einer Epstein-Barr-Virusinfektion.

25. Verwendung einer Verbindung nach mindestens einem der Ansprüche 1 bis 5 bei der Herstellung eines Arzneimittels zur Behandlung oder Prophylaxe einer Hepatitis B-Virusinfektion.

26. Pharmazeutische Zusammensetzung, umfassend als Wirkstoff eine Verbindung nach mindestens einem der Ansprüche 1 bis 5, zusammen mit einem pharmazeutisch annehmbaren Träger.

27. Pharmazeutische Zusammensetzung nach Anspruch 26, welche zur Verwendung bei der Behandlung oder Prophylaxe einer Herpes simplex-Virusinfektion angepaßt ist.

28. Pharmazeutische Zusammensetzung nach Anspruch 26, welche zur Verwendung bei der Behandlung oder Prophylaxe einer Varicella zoster-Virusinfektion angepaßt ist.

29. Pharmazeutische Zusammensetzung nach Anspruch 26, welche zur Verwendung bei der Behandlung oder Prophylaxe einer Zytomegalovirusinfektion angepaßt ist.

30. Pharmazeutische Zusammensetzung nach mindestens einem der Ansprüche 26 bis 29, welche für die orale Verabreichung angepaßt ist.

31. Pharmazeutische Zusammensetzung nach Anspruch 30 in Form einer Tablette oder Kapsel.

32. Pharmazeutische Zusammensetzung nach Anspruch 31, wobei die Tablette oder Kapsel 10 bis 1000 mg einer Verbindung nach mindestens einem der Ansprüche 1 bis 5 enthält.

33. Pharmazeutische Zusammensetzung nach Anspruch 31, wobei die Tablette oder Kapsel 20 bis 500 mg einer Verbindung nach mindestens einem der Ansprüche 1 bis 5 enthält.

34. Verwendung der Verbindungen nach den Ansprüchen 1 bis 5 in Kombination mit Zidovudin bei der Herstellung eines Arzneimittels zur Verwendung bei der Behandlung oder Prophylaxe einer HIV-Infektion.

35. Verfahren zur Herstellung einer Verbindung der Formel (I) 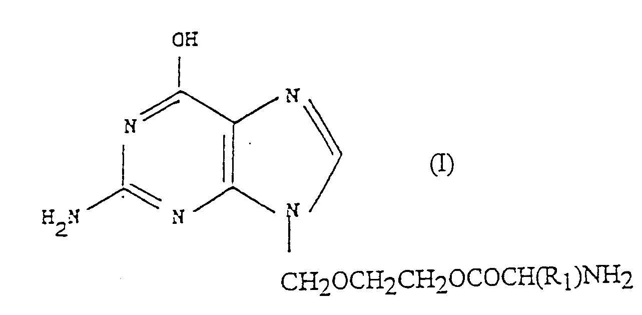 worin R₁ eine Gruppe der Formel -CH[CH₃]₂ bedeutet und die Estergruppe (OCOCH(R₁)NH₂) in der L-Konfiguration vorliegt, oder eines pharmazeutisch annehmbaren Salzes davon, umfassend:
a) Umsetzen einer Verbindung der Formel (II) 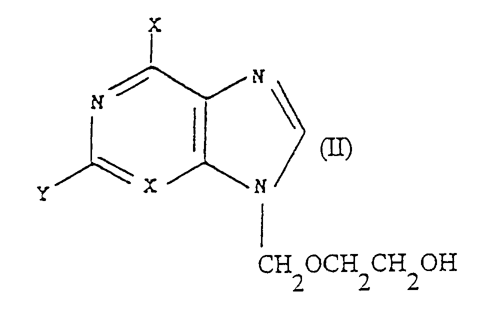 worin X eine wahlweise geschützte Hydroxygruppe ist und Y eine wahlweise geschützte Aminogruppe ist, mit einem wahlweise geschützten L-Valin oder einem funktionellen Äquivalent davon;
b) Umwandeln einer Verbindung der Formel (III) 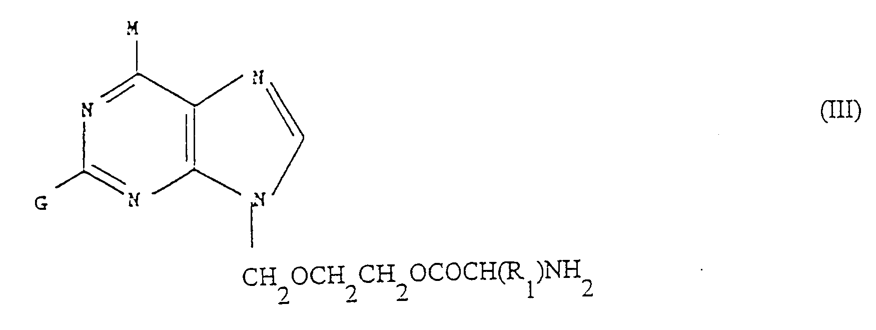 worin R₁ wie oben definiert ist; M eine Hydroxygruppe bedeutet und G ein Atom oder eine Gruppe bedeutet, welche durch eine Aminogruppe ersetzt oder in diese überführt werden kann; oder worin G eine Aminogruppe bedeutet und M ein Atom oder eine Gruppe bedeutet, welche durch eine Hydroxygruppe ersetzt oder in diese überführt werden kann, in eine Verbindung der Formel (I) oder ein pharmazeutisch annehmbares Salz davon; oder
c) Umsetzen einer Verbindung der Formel (IV) 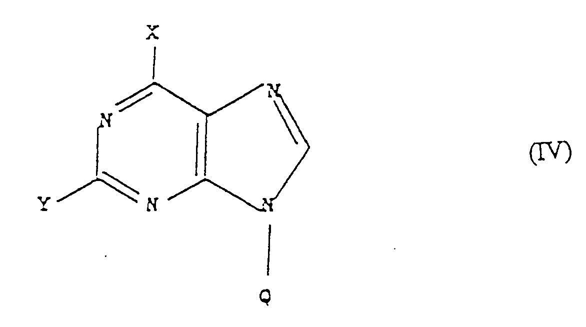 worin X und Y wie oben definiert sind und Q ein Abgangsatom oder eine Abgangsgruppe ist, mit einer Verbindung der Formel (V)
ACH₂OCH₂CH₂OCOCH(R₁)R₂ (V)
worin R₁ wie oben definiert ist, A eine Abgangsgruppe oder ein Abgangsatom bedeutet und R₂ eine wahlweise geschützte Aminogruppe ist; und wahlweise Bewirken einer oder mehrerer der folgenden Umwandlungen in einer erwünschten Reihenfolge:
i) Entfernung irgendwelcher Schutzgruppen;
ii) wenn das resultierende Produkt eine Verbindung der Formel (I) ist, Umwandlung dieser Verbindung in ein pharmazeutisch annehmbares Salz davon; und
iii) wenn das resultierende Produkt ein pharmazeutisch annehmbares Salz einer Verbindung der Formel (I) ist, Umwandlung des Salzes in die Stammverbindung.

## Patentansprüche (Patentansprüche für folgende(n) Vertragsstaat(en): ES, GR)

1. Verfahren zur Herstellung einer Verbindung der Formel (I) 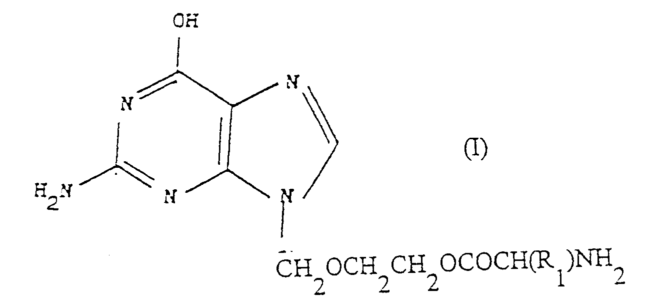 worin R₁ eine Gruppe der Formel -CH[CH₃]₂ bedeutet und die Estergruppe (OCOCH(R₁)NH₂) in der L-Konfiguration vorliegt, oder eines pharmazeutisch annehmbaren Salzes davon, umfassend:
a) Umsetzen einer Verbindung der Formel (II) 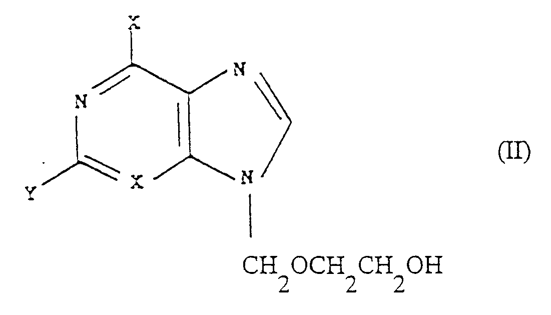 worin X eine wahlweise geschützte Hydroxygruppe ist und Y eine wahlweise geschützte Aminogruppe ist, mit einem wahlweise geschützten L-Valin oder L-Isoleucin oder einem funktionellen Äquivalent davon;
b) Umwandeln einer Verbindung der Formel (III) 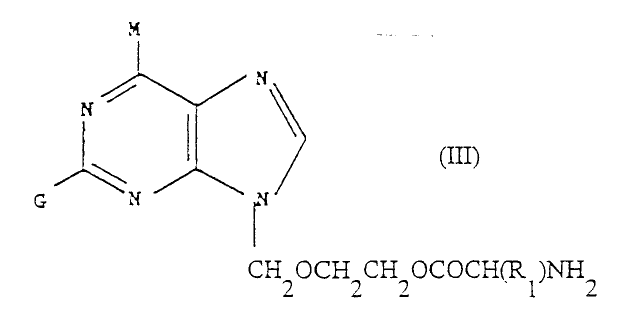 worin R₁ wie oben definiert ist; M eine Hydroxygruppe bedeutet und G ein Atom oder eine Gruppe bedeutet, welche durch eine Aminogruppe ersetzt oder in diese überführt werden kann; oder worin G eine Aminogruppe bedeutet und M ein Atom oder eine Gruppe bedeutet, welche durch eine Hydroxygruppe ersetzt oder in diese überführt werden kann, in eine Verbindung der Formel (I) oder ein pharmazeutisch annehmbares Salz davon; oder
c) Umsetzen einer Verbindung der Formel (IV) 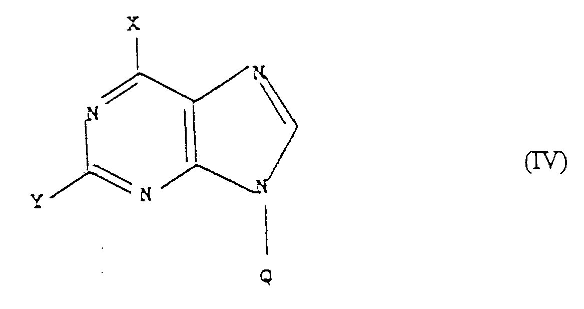 worin X und Y wie oben definiert sind und Q ein Abgangsatom oder eine Abgangsgruppe ist, mit einer Verbindung der Formel (V)
ACH₂OCH₂CH₂OCOCH(R₁)R₂ (V)
worin R₁ wie oben definiert ist, A eine Abgangsgruppe oder ein Abgangsatom bedeutet und R₂ eine wahlweise geschützte Aminogruppe ist; und wahlweise Bewirken einer oder mehrerer der folgenden Umwandlungen in einer erwünschten Reihenfolge:
i) Entfernung irgendwelcher Schutzgruppen;
ii) wenn das resultierende Produkt eine Verbindung der Formel (I) ist, Umwandlung dieser Verbindung in ein pharmazeutisch annehmbares Salz davon; und
iii) wenn das resultierende Produkt ein pharmazeutisch annehmbares Salz einer Verbindung der Formel (I) ist, Umwandlung des Salzes in die Stammverbindung.

2. Verfahren nach Anspruch 1, wobei die Verbindung der Formel (I) 2-((2-Amino-1,6-dihydro-6-oxo-9H-purin-9-yl)methoxy)ethyl-L-valinat ist.

3. Verfahren zur Herstellung eines Salzes einer Verbindung nach den Ansprüchen 1 oder 2, umfassend das Mischen einer Verbindung der Formel (I) mit einer Säure zur Bildung eines Salzes.

4. Verfahren nach Anspruch 3, wobei die Säure aus der Chlorwasserstoffsäure, Schwefelsäure, Phosphorsäure, Maleinsäure, Fumarsäure, Zitronensäure, Weinsäure, Milchsäure, Essigsäure und p-Toluolsulfonsäure umfassenden Gruppe gewählt wird.

5. Verfahren nach Anspruch 4, wobei die Säure Chlorwasserstoffsäure ist.

6. Verfahren nach mindestens einem der vorangehenden Ansprüche, wobei die Verbindung der Formel (I) 2-((2-Amino-1,6-dihydro-6-oxo-9H-purin-9-yl)methoxy)ethyl-L-valinathydrochloridmonohydrat ist.

7. Verfahren nach mindestens einem der vorangehenden Ansprüche, wobei die Schutzgruppe beim Verfahren A nach Anspruch 1 N,N'-Dicyclohexylcarbodiimid ist.

8. Verfahren nach Anspruch 7, wobei dieses in Pyridin oder Dimethylformamid durchgeführt wird.

9. Verfahren nach Anspruch 7 oder 8, wobei dieses in Gegenwart einer katalytischen Base durchgeführt wird.

10. Verfahren nach Anspruch 9, wobei die katalytische Base 4-Dimethylaminopyridin ist.

11. Verfahren nach mindestens einem der vorangehenden Ansprüche, wobei das funktionelle Derivat des Verfahrens A ein Säurehalogenid oder Säureanhydrid ist, wobei die Aminogruppe des L-Valins geschützt ist.

12. Verfahren nach Anspruch 11, wobei die Aminoschutzgruppe ein Acetyl oder ein Aryloxycarbonyl ist.

13. Verfahren gemäß Verfahren B nach mindestens einem der Ansprüche 1 bis 6, wobei G eine Azidgruppe bedeutet, welche durch katalytische Hydrierung zu einer Aminogruppe reduziert wird.

14. Verfahren gemäß Verfahren B nach mindestens einem der Ansprüche 1 bis 6, wobei G ein Halogenatom oder eine Alkylthio- oder Alkylsulfonylgruppe bedeutet, welche in eine Azidgruppe überführt wird, welche wiederum durch katalytische Hydrierung in eine Aminogruppe überführt wird.

15. Verfahren nach den Ansprüchen 13 oder 14, wobei M eine Aminogruppe ist und durch Behandlung mit einem deaminierenden Enzym in eine Hydroxygruppe umgewandelt wird.

16. Verfahren gemäß Verfahren C nach mindestens einem der Ansprüche 1 bis 6, wobei Q ein Wasserstoffatom, eine Acylgruppe oder eine Tri-C₁₋₄-alkylsilylgruppe bedeutet.

17. Verfahren nach Anspruch 16, wobei Q eine Acetyl- oder Benzoylgruppe bedeutet.

18. Verfahren nach den Ansprüchen 15 oder 16, wobei A ein Halogenatom oder eine Acyloxygruppe bedeutet, wobei der Acylteil eine C₁₋₄-Alkanoyl- oder eine Aroylgruppe ist.

19. Verfahren nach mindestens einem der Ansprüche 16 bis 18, wobei R₂ eine C₁₋₄-Alkanoyl-Aryloxycarbonyl- oder eine Azidogruppe bedeutet.

20. Verfahren nach den Ansprüchen 16 bis 19, wobei dieses in Dimethylformamid oder Hexamethylphosphoramid in Gegenwart einer Base bewirkt wird.

21. Verfahren nach den Ansprüchen 16 bis 18, wobei eine thermische Kondensation durch Erwärmen der Verbindungen der Formel (IV) und (V) in Gegenwart einer katalytischen Menge einer starken Säure bewirkt wird.

22. Verfahren nach mindestens einem der vorangehenden Ansprüche, wobei die Schutzgruppen durch Hydrolyse, Solvolyse oder Hydrogenolyse entfernt werden.

23. Verfahren nach Anspruch 22, wobei, wenn die Schutzgruppe ein Aryloxycarbonyl ist, Hydrogenolyse zu deren Entfernung angewandt wird.

24. Verfahren zur Herstellung einer pharmazeutischen Zubereitung, enthaltend eine Verbindung der Formel (I) wie in Anspruch 1 definiert oder ein pharmazeutisch annehmbares Salz davon, umfassend die Herstellung einer Verbindung der Formel (I) oder eines pharmazeutisch annehmbaren Salzes davon durch:
a) Umsetzen einer Verbindung der Formel (II) 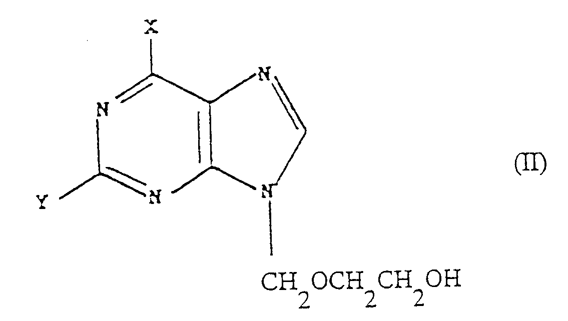 worin X eine wahlweise geschützte Hydroxygruppe ist und Y eine wahlweise geschützte Aminogruppe ist, mit einem wahlweise geschützten L-Valin oder einem funktionellen Äquivalent davon;
b) Umwandeln einer Verbindung der Formel (III) 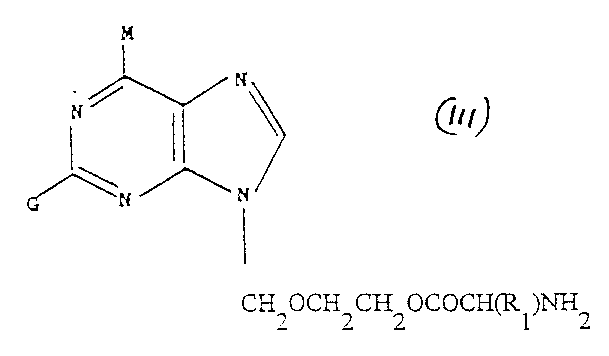 worin R₁ wie in Anspruch 1 definiert ist; M eine Hydroxygruppe bedeutet und G ein Atom oder eine Gruppe bedeutet, welche durch eine Aminogruppe ersetzt oder in diese überführt werden kann; oder worin G eine Aminogruppe bedeutet und M ein Atom oder eine Gruppe bedeutet, welche durch eine Hydroxygruppe ersetzt oder in diese überführt werden kann, in eine Verbindung der Formel (I) oder ein pharmazeutisch annehmbares Salz davon; oder
c) Umsetzen einer Verbindung der Formel (IV) 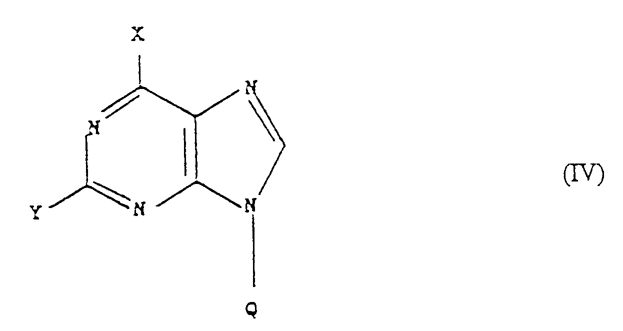 worin X und Y wie oben definiert sind und Q ein Abgangsatom oder eine Abgangsgruppe ist, mit einer Verbindung der Formel (V)
ACH₂OCH₂CH₂OCOCH(R₁)R₂ (V)
worin R₁ wie in Anspruch 1 definiert ist, A eine Abgangsgruppe oder ein Abgangsatom bedeutet und R₂ eine wahlweise geschützte Aminogruppe ist; und wahlweise Bewirken einer oder mehrerer der folgenden Umwandlungen in einer erwünschten Reihenfolge:
i) Entfernung irgendwelcher Schutzgruppen;
ii) wenn das resultierende Produkt eine Verbindung der Formel (I) ist, Umwandlung dieser Verbindung in ein pharmazeutisch annehmbares Salz davon; und
iii) wenn das resultierende Produkt ein pharmazeutisch annehmbares Salz einer Verbindung der Formel (I) ist, Umwandlung des Salzes in die Stammverbindung;
und Zusammenbringen der resultierenden Verbindung mit einem pharmazeutisch annehmbaren Träger.

## Revendications (Revendications pour l'(les) Etat(s) contractant(s) suivant(s): AT, BE, CH, DE, FR, GB, IT, LI, LU, NL, SE)

1. L-valinate de 2-((2-amino-1,6-dihydro-6-oxo-9H-purine-9-yl)méthoxy)éthyle ou sel pharmaceutiquement acceptable de celui-ci.

2. Sel d'un composé selon la revendication 1 où le sel est un sel d'addition d'acide.

3. Sel d'un composé selon la revendication 2 qui est dérivé de l'acide chlorhydrique, sulfurique, phosphorique, maléique, fumarique, citrique, tartrique, lactique, acétique ou p-toluènesulfonique.

4. Chlorhydrate de L-valinate de 2-((2-amino-1,6-dihydro-6-oxo-9H-purine-9-yl)méthoxy)éthyle.

5. L-valinate de 2-((2-amino-1,6-dihydro-6-oxo-9H-purine-9-yl)méthoxy)éthyle.

6. Composé selon l'une quelconque des revendications précédentes, à usage médical thérapeutique.

7. Composé selon la revendication 6, utilisable pour le traitement curatif ou préventif d'une infection virale.

8. Composé selon la revendication 7 utilisable pour le traitement curatif ou préventif d'une infection par le virus de l'herpès simplex.

9. Composé selon la revendication 8 utilisable pour le traitement curatif ou préventif d'une infection par le virus de l'herpès simplex de type 1.

10. Composé selon la revendication 7 utilisable pour le traitement curatif ou préventif d'une infection par le virus de la varicelle-zona.

11. Composé selon la revendication 10 utilisable pour le traitement curatif ou préventif de la varicelle.

12. Composé selon la revendication 10 utilisable pour le traitement curatif ou préventif du zona.

13. Composé selon la revendication 7 utilisable pour le traitement curatif ou préventif d'une infection par le cytomégalovirus.

14. Composé selon la revendication 7 utilisable pour le traitement curatif ou préventif d'une infection par le virus d'Epstein-Barr.

15. Composé selon la revendication 7 utilisable pour le traitement curatif ou préventif d'une infection par le virus de l'hépatite B.

16. Utilisation d'un composé selon l'une quelconque des revendications 1 à 5 dans la préparation d'un médicament pour le traitement curatif ou préventif d'une infection virale.

17. Utilisation d'un composé selon l'une quelconque des revendications 1 à 5 dans la préparation d'un médicament pour le traitement curatif ou préventif d'une infection virale herpétique.

18. Utilisation d'un composé selon l'une quelconque des revendications 1 à 5 dans la préparation d'un médicament pour le traitement curatif ou préventif d'une infection par le virus de l'herpès simplex.

19. Utilisation d'un composé selon la revendication 18 dans la préparation d'un médicament pour le traitement curatif ou préventif d'une infection par l'herpès simplex de type 1.

20. Utilisation d'un composé selon l'une quelconque des revendications 1 à 5 dans la préparation d'un médicament pour le traitement curatif ou préventif d'une infection par le virus de la varicelle-zona.

21. Utilisation d'un composé selon la revendication 20 dans la préparation d'un médicament pour le traitement curatif ou préventif de la varicelle.

22. Utilisation d'un composé selon la revendication 20 dans la préparation d'un médicament pour le traitement curatif ou préventif du zona.

23. Utilisation d'un composé selon l'une quelconque des revendications 1 à 5 dans la préparation d'un médicament pour le traitement curatif ou préventif d'une infection par le cytomégalovirus.

24. Utilisation d'un composé selon l'une quelconque des revendications 1 à 5 dans la préparation d'un médicament pour le traitement curatif ou préventif d'une infection par le virus d'Epstein-Barr.

25. Utilisation d'un composé selon l'une quelconque des revendications 1 à 5 dans la préparation d'un médicament pour le traitement curatif ou préventif d'une infection par le virus de l'hépatite B.

26. Composition pharmaceutique comprenant un constituant actif selon l'une quelconque des revendications 1 à 5 avec un excipient pharmaceutiquement acceptable.

27. Composition pharmaceutique selon la revendication 26, convenant à une utilisation dans le traitement curatif ou préventif d'une infection par le virus de l'herpès simplex.

28. Composition pharmaceutique selon la revendication 26, convenant à une utilisation dans le traitement curatif ou préventif d'une infection par le virus de la varicelle-zona.

29. Composition pharmaceutique selon la revendication 26, convenant à une utilisation dans le traitement curatif ou préventif d'une infection par le cytomégalovirus.

30. Composition pharmaceutique selon l'une quelconque des revendications 26 à 29 convenant pour une administration par voie orale.

31. Composition pharmaceutique selon la revendication 30, sous la forme d'un comprimé ou d'une capsule.

32. Composition pharmaceutique selon la revendication 31, dans laquelle le comprimé ou la capsule contient de 10 à 1000 mg d'un composé selon l'une quelconque des revendications 1 à 5.

33. Composition pharmaceutique selon la revendication 31, dans laquelle le comprimé ou la capsule contient de 20 à 500 mg d'un composé selon l'une quelconque des revendications 1 à 5.

34. Utilisation des composés selon les revendications 1 à 5 en combinaison avec la zidovudine dans la préparation d'un médicament utilisable dans le traitement curatif ou préventif d'une infection par le VIH.

35. Procédé de préparation d'un composé de formule (I) 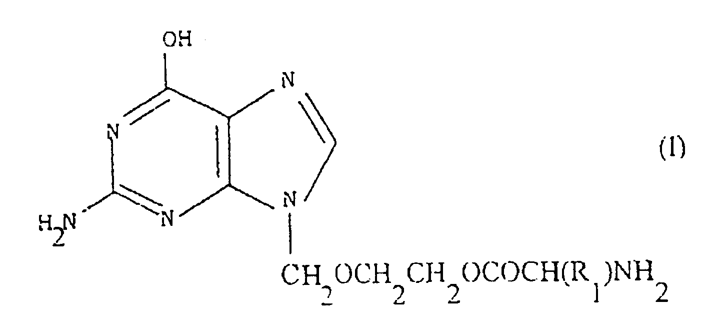 dans laquelle R₁ représente un groupement de formule -CH[CH₃]₂, le groupement ester (OCOCH(R₁)NH₂) étant en configuration L, ou un sel pharmaceutiquement acceptable de celui-ci, procédé qui comprend les étapes consistant à :
a) faire réagir un composé de formule (II) 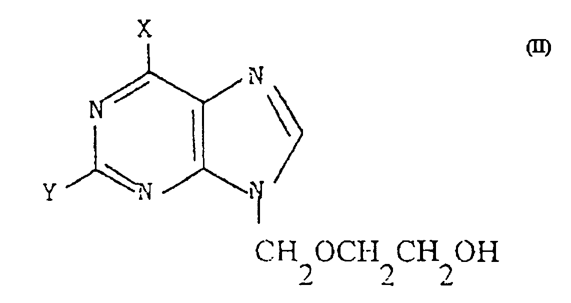 où X est un groupement hydroxy éventuellement protégé, et Y un groupement amino éventuellement protégé avec une L-valine éventuellement protégée, ou un équivalent fonctionnel de celles-ci ;
b) convertir un composé de formule (III) 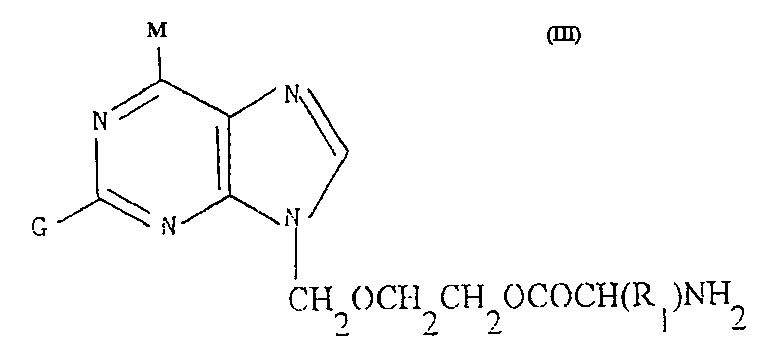 (où R₁ est tel que défini ci-dessus; et M représente un groupement hydroxy et G représente un atome ou un groupement qui peut être remplacé par, ou converti en, un groupement amino ; ou bien G représente un groupement amino et M représente un atome ou un groupement qui peut être remplacé par, ou converti en, un groupement hydroxy) en un composé de formule (I) ou un sel pharmaceutiquement acceptable de celui-ci ; ou bien
c) faire réagir un composé de formule (IV) 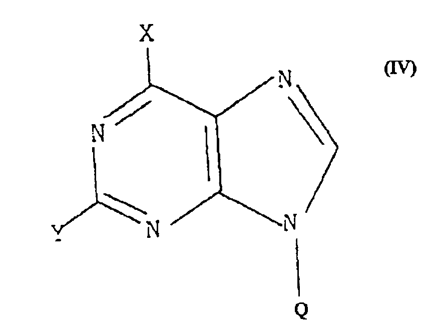 (où X et Y sont tels que définis ci-dessus et Q représente un atome ou un groupement partant) avec un composé de formule (V)
ACH₂OCH₂CH₂OCOCH(R₁)R₂ (V)
dans laquelle R₁ est tel que défini ci-dessus, A représente un groupement ou un atome partant et R₂ est un groupement amino éventuellement protégé ; et en effectuant éventuellement une ou plusieurs des conversion suivantes, dans l'ordre quelconque désiré :
i) éliminer tout groupement de protection ;
ii) lorsque le produit résultant est un composé de formule (I), convertir ledit composé en un sel pharmaceutiquement acceptable de celui-ci ; et
iii) lorsque le produit résultant est un sel pharmaceutiquement acceptable d'un composé de formule (I), convertir ledit sel en ledit composé.

## Revendications (Revendications pour l'(les) Etat(s) contractant(s) suivant(s): ES, GR)

1. Procédé de préparation d'un composé de formule (I) 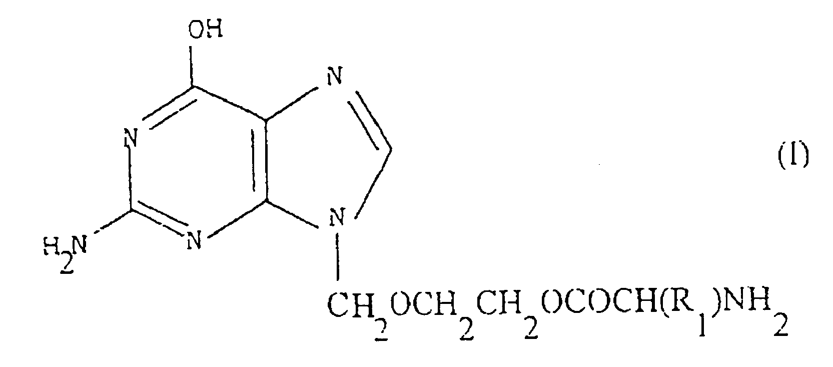 dans laquelle R₁ représente un groupement de formule -CH[CH₃]₂, le groupement ester (-OCOCH(R₁)NH₂) étant en configuration L, ou un sel pharmaceutiquement acceptable de celui-ci, procédé qui comprend les étapes consistant à :
a) faire réagir un composé de formule (II) 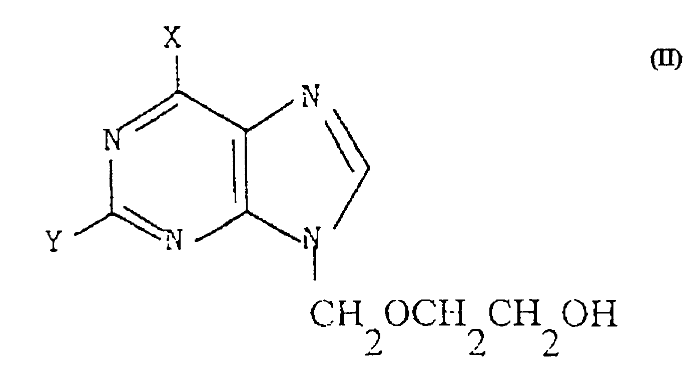 où X est un groupement hydroxy éventuellement protégé, et Y un groupement amino éventuellement protégé, avec une L-valine éventuellement protégée ou un équivalent fonctionnel de celle-ci ;
b) convertir un composé de formule (III) 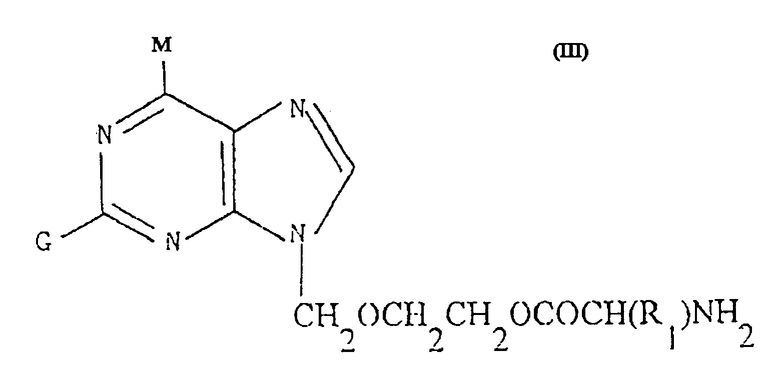 (où R₁ est tel que défini dans la revendication 1 ; M représente un groupement hydroxy et G représente un atome ou un groupement qui peut être remplacé par ou converti en un groupement amino ; ou bien G représente un groupement amino et M représente un atome ou un groupement qui peut être remplacé par, ou converti en, un groupement hydroxy), en un composé de formule (I) ou un sel pharmaceutiquement acceptable de celui-ci ; ou bien
c) faire réagir un composé de formule (IV) 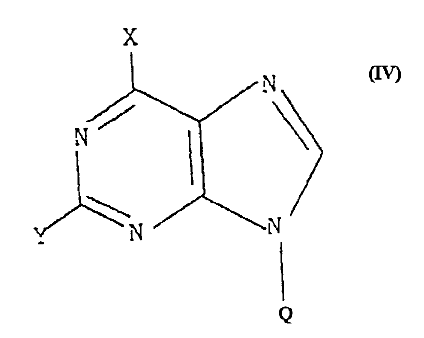 (où X et Y sont tels que définis ci-dessus et Q représente un atome ou un groupement partant) avec un composé de formule (V)
ACH₂OCH₂CH₂OCOCH(R₁)R₂ (V)
dans laquelle R₁ est tel que défini dans la revendication 1, A représente un groupement ou un atome partant et R₂ est un groupement amino éventuellement protégé ; et en effectuant éventuellement une ou plusieurs des conversion suivantes, dans l'ordre quelconque désiré :
i) éliminer tout groupement de protection ;
ii) lorsque le produit résultant est un composé de formule (I), convertir ledit composé en un sel pharmaceutiquement acceptable de celui-ci ; et
iii) lorsque le produit résultant est un sel pharmaceutiquement acceptable d'un composé de formule (I), convertir ledit sel en ledit composé.

2. Procédé selon la revendication 1 où le composé de formule (I) est le L-valinate de 2-((2-amino-1,6-dihydro-6-oxo-9H-purine-9-yl)méthoxy)éthyle

3. Procédé de préparation d'un sel d'un composé selon les revendications 1 ou 2 qui comprend le mélange d'un composé de formule (I) avec un acide pour former un sel.

4. Procédé selon la revendication 3 dans lequel ledit acide est choisi dans le groupe constitué de l'acide chlorhydrique, sulfurique, phosphorique, maléique, fumarique, citrique, tartrique, lactique, acétique et p-toluènesulfonique.

5. Procédé selon la revendication 4 dans lequel ledit acide est l'acide chlorhydrique.

6. Procédé selon l'une quelconque des revendications précédentes dans lequel le composé de formule (I) est le chlorhydrate monohydraté de L-valinate de 2-((2-amino-1,6-dihydro-6-oxo-9H-purine-9-yl)méthoxy) éthyle.

7. Procédé selon l'une quelconque des revendications précédentes dans lequel le groupe de protection dans le procédé A de la revendication 1 est le N,N'-dicyclohexylcarbodiimide.

8. Procédé selon la revendication 7 qui est réalisé dans la pyridine ou le diméthylformamide.

9. Procédé selon la revendication 7 ou 8 qui est réalisé en présence d'un catalyseur basique.

10. Procédé selon la revendication 9 dans lequel le catalyseur basique est la 4-diméthylaminopyridine.

11. Procédé selon l'une quelconque des revendications précédentes dans lequel le dérivé fonctionnel du procédé A est un halogénure d'acide ou un anhydride d'acide dans lequel le groupement amino de la L-valine est protégé.

12. Procédé selon la revendication 11 dans lequel le groupement protégeant le groupement amino est un acétyle ou un aryloxycarbonyle.

13. Procédé selon le procédé B de l'une quelconque des revendications 1 à 6 précédentes dans lequel G représente un groupement azoture qui est réduit en groupement amino par hydrogénation catalytique.

14. Procédé selon le procédé B de l'une quelconque des revendications 1 à 6, dans lequel G représente un atome d'halogène ou un groupement alkylthio ou alkylsulfonyle qui est converti en un groupement azoture qui, à son tour, est converti en groupement amino par hydrogénation catalytique.

15. Procédé selon la revendication 13 ou 14 dans lequel M est un groupement amino et est converti en un groupement hydroxy par désamination enzymatique.

16. Procédé selon le procédé C de l'une quelconque des revendications 1 à 6 dans lequel Q représente un atome d'hydrogène, un groupement acyle ou un groupement tri-alkylC₁₋₄-silyle.

17. Procédé selon la revendication 16 dans lequel Q représente un groupement acétyle ou benzoyle.

18. Procédé selon la revendication 15 ou 16 dans lequel A représente un halogène ou un groupement acyloxy dans lequel l'acyle est un groupement alkanoyle en C₁₋₄ ou aroyle.

19. Procédé selon l'une quelconque des revendications 16 à 18 dans lequel R₂ représente un groupement alkanoyle en C₁₋₄, aryloxycarbonyle ou azido.

20. Procédé selon les revendications 16 à 19 qui est réalisé dans du diméthylformamide ou de l'hexaméthylphosphoramide en présence d'une base.

21. Procédé selon les revendications 16 à 18 dans lequel on effectue une condensation thermique en chauffant les composés de formule (IV) et (V) en présence d'une quantité catalytique d'un acide fort.

22. Procédé selon l'une quelconque des revendications précédentes dans lequel les groupements protecteurs sont éliminés par hydrolyse, solvolyse ou hydrogénolyse.

23. Procédé selon la revendication 22, dans lequel on utilise l'hydrogénolyse pour éliminer le groupement protecteur lorsque celui-ci est un aryloxycarbonyle.

24. Procédé de préparation d'une formulation pharmaceutique contenant le composé de formule (I), tel que défini dans la revendication 1 ou un sel pharmaceutiquement acceptable de celui-ci, qui comprend la préparation d'un composé de formule (I) ou un sel pharmaceutiquement acceptable de celui-ci suivant les étapes consistant à :
a) faire réagir un composé de formule (II) 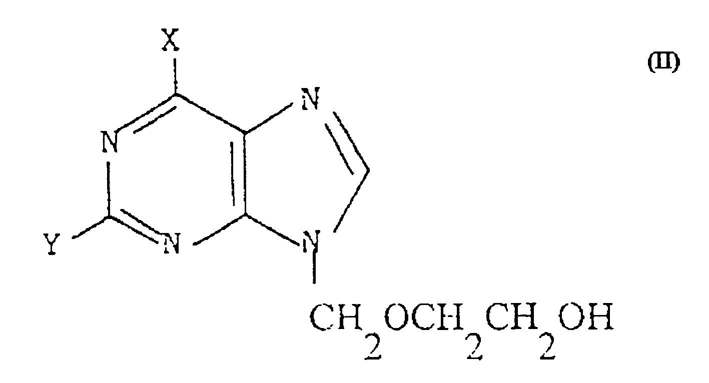 où X est un groupement hydroxy éventuellement protégé, et Y un groupement amino éventuellement protégé, avec une L-valine éventuellement protégée ou un équivalent fonctionnel de celle-ci ;
b) convertir un composé de formule (III) 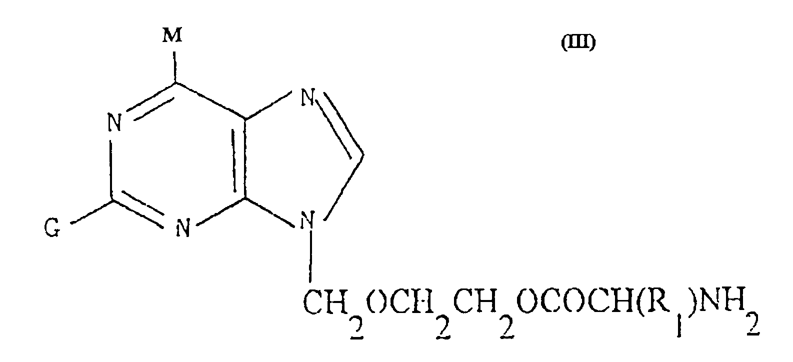 (où R₁ est tel que défini dans la revendication 1 ; M représente un groupement hydroxy et G représente un atome ou un groupement qui peut être remplacé par, ou converti en, un groupement amino ; ou bien G représente un groupement amino et M représente un atome ou un groupement qui peut être remplacé par, ou converti en, un groupement hydroxy) en un composé de formule (I) ou un sel pharmaceutiquement acceptable de celui-ci ; ou bien
c) faire réagir un composé de formule (IV) 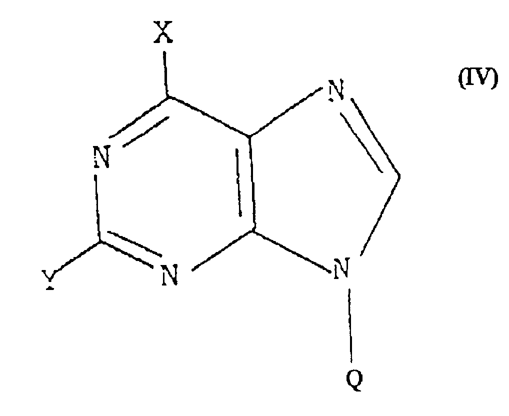 (où X et Y sont tels que définis ci-dessus et Q représente un atome ou un groupement partant) avec un composé de formule (V)
ACH₂OCH₂CH₂OCOCH(R₁)R₂ (V)
dans laquelle R₁ est tel que défini ci-dessus, A représente un groupement ou un atome partant et R₂ est un groupement amino éventuellement protégé ; et en effectuant éventuellement une ou plusieurs des conversion suivantes, dans l'ordre quelconque désiré :
i) éliminer tout groupement de protection ;
ii) lorsque le produit résultant est un composé de formule (I), convertir ledit composé en un sel pharmaceutiquement acceptable de celui-ci ; et
iii) lorsque le produit résultant est un sel pharmaceutiquement acceptable d'un composé de formule (I), convertir ledit sel en ledit composé.
et associer le composé résultant avec un excipient pharmaceutiquement acceptable.
